# EUROPEAN PATENT APPLICATION

(11) **EP 3 345 592 A1**
(43) Date of publication of application: **11.07.2018**
(21) Application number: 18150904.3
(22) Date of filing: 09.01.2018
(51) Int. Cl.: A61K 9/00, A61K 47/12, A61K 47/18, A61K 31/407, A61P 29/00

(54) **ENHANCED STABILITY KETOROLAC FORMULATIONS AND METHODS AND DEVICES FOR USE WITH SAME**

(30) Priority: 09.01.2017 US 201762443856 P
(71) Applicant: Hurrey, Michael Laird, San Ramon, CA 94583 (US); Noymer, Peter, Los Gatos, CA 95032 (US)
(72) Inventor: Hurrey, Michael Laird, San Ramon, CA 94583 (US); Noymer, Peter, Los Gatos, CA 95032 (US)
(74) Representative: Fairbairn, Angus Chisholm

(57) **Abstract**

Provided is a unit dosage form of Ketorolac in a sterile fluid composition formulated for continuous subcutaneous delivery via body-worn infusion pump assembly, wherein said Ketorolac is present at a concentration of between **40** mg/mL and **240** mg/mL formulated for single use delivery in a volume not to exceed 1-10 mL, wherein said formulation is ethanol-free. Uses of same, body-worn infusion pump assemblies containing same and containers containing same for incorporation within a body-worn infusion pump assembly for a dose-sparing formulation comprising Ketorolac are also provided.

## Description

### BACKGROUND OF THE INVENTION

Ketorolac is a nonsteroidal anti-inflammatory drug and an inhibitor of prostaglandin synthesis, and thus possesses anti-inflammatory, analgesic and antipyretic activities.

The chemical name of Ketorolac is (±)-5-benzoyl-2,3-dihydro-1H-pyrrolizine-1-carboxylic acid, compound with 2-amino-2-(hydroxymethyl)-1,3-propanediol.

Pharmaceutical formulations with analgesic and antipyretic action, containing Ketorolac have been described, as has Ketorolac use for therapy in pain management, ocular pain treatment, periodontal affection treatments, and for the treatment of carcinomas of the scaly cells in the oral cavity or of the oropharynx.

Ketorolac formulations for oral, parenteral and topical administration have been described, as have several pharmaceutical forms, including tablets, suppository, pills, capsules, powder, solutions, suspensions, emulsions, creams, lotions and unguents.

Other formulations containing Ketorolac have been described, allowing for the application of Ketorolac through dentifrices, and including solutions for mouthwash and spray for oral cavity or dental solutions.

Nasal sprays containing Ketorolac have been described, as have transdermal formulations.

Formulations containing Ketorolac providing rapid anti-inflammatory, antipyretic and analgesic action include those via injection, yet the same are associated with illness and discomfort to the patient.

Given the importance in the use of Ketorolac as an analgesic, it is clear that compromising the speed of Ketorolac action is significantly undesirable, and the side effects associated with injectable forms of Ketorolac highlight the importance and need for providing a more efficacious and/or more tolerable Ketorolac treatment for patients.

### SUMMARY OF THE INVENTION

It is an object of this invention to provide a more efficacious and/or more comfortable, consistent and reliable Ketorolac treatment for patients in need of same, which in some aspects yields a formulation that is much more stable over time.

In some aspects of this invention, there is provided a Ketorolac-containing formulation adapted to contain specific stabilizers in an ethanol free setting and/or sodium chloride free setting, which provides for enhanced formulation stability. In some aspects, such formulation is characterized by enhanced stability even when the formulation is stored at room temperature, or in some embodiments, when the formulation is stored at higher temperatures, such as, for example, that experienced in the United States in various states, in various seasons outdoors, etc. In some aspects, such enhanced stability is in addition to other advantages as described herein, for example, for example, in terms of a dose sparing effect, and others, as described herein.

In some aspects, this invention provides a formulation of Ketorolac for subcutaneous administration or for continuous subcutaneous administration, which formulation has a maximal volume of infusion which does not exceed 10 mL. In some aspects, the delivery volume is 1 mL per day for a normal adult and in some embodiments, the delivery volume is from 0.5-5 mL per day. In some embodiments the delivery volume is from 1-3 mL per day.

In some embodiments, this invention provides a sterile fluid composition for continuous subcutaneous delivery via infusion pump assembly, said composition comprising Ketorolac at a concentration of between **40** mg/mL and **240** mg/mL in a volume not to exceed 2-5 mL.

In some embodiments, this invention provides a unit dosage form of Ketorolac in a sterile fluid composition formulated for continuous subcutaneous delivery via infusion pump assembly, wherein said Ketorolac is present at a concentration of between **40** mg/mL and **240** mg/mL formulated for single use delivery in a volume not to exceed 2-5 mL.

In some aspects, this invention provides a formulation of Ketorolac for continuous subcutaneous administration, which formulation delivers 240 mg/ml.

In some aspects, this invention provides a formulation of Ketorolac for continuous subcutaneous administration, which formulation delivers 120 mg/ml.

In some aspects, this invention provides a formulation of Ketorolac for continuous subcutaneous administration, which formulation delivers 90 mg/ml.

In some aspects, this invention provides a formulation of Ketorolac for continuous subcutaneous administration, which formulation delivers 60 mg/ml.

Such formulations will be characterized by enhanced stability even at room temperature or higher temperatures, for example, reducing or abrogating a need for refrigeration of the formulation.

In some aspects, in addition to the enhanced stability, the Ketorolac formulations of this invention provide for a further dose-sparing effect. In some aspects, the adult daily dosage may be provided as from 60 - 120 mg/day. In some aspects, the adult daily dosage may be provided as from 60 - 90 mg/day.

In some aspects, the adult daily dosage typically used for normal weight, non-elderly patients is 120 mg/day, and a daily dosage 20% - 40% less than same can be provided with the formulations and delivery system of this invention. For example, in some embodiments, formulations providing a daily dosage of 80-90 mg/day are envisioned. In some aspects, the daily dosage for elderly, or underweight patients typically used is 60 mg/day, which as well may be reduced by from 20% - 40% less, by using the formulations and delivery systems of this invention. For example, in some embodiments, formulations providing a daily dosage of 40-45 mg/day are envisioned.

In some aspects, the invention provides for a single use container comprising the described Ketorolac formulation for delivery by infusion pump, which formulation provides for fewer side effects experienced by the patient in use of same.

In some aspects, the invention provides for a single use container comprising the described Ketorolac formulation for delivery by infusion pump, which formulation provides for greater formulation stability and/or less oxidation as compared to other formulations of Ketorolac in use to date.

In some aspects, this invention provides a method of providing analgesia to a patient in need thereof comprising administering a Ketorolac composition of this invention formulated for subcutaneous administration to maintain an analgesic response in said patient. According to this aspect, and in some embodiments, such composition is formulated for continuous subcutaneous administration. According to this aspect, and in some embodiments, such composition achieves analgesia in a patient continuously during administration, and in some embodiments, effective analgesia is achieved with fewer side effects associated with use of other means of administering Ketorolac. In some aspects, diminishing or abrogating certain side effects may in turn lead to greater analgesic effect, as well.

In some embodiments such method results in the ability to achieve the desired analgesic, anti-inflammatory, anti-pyretic effect or combination thereof in the patient with a reduced dosage or sustained dosage over a significantly prolonged period of time, without at least some of the adverse effects previously reported with Ketorolac use.

In some embodiments, such method results in less of a need to administer higher Ketorolac dosages over time and in some embodiments, such method results in a less frequent need for adjustment of a dosage provided to such subject, over time, and in some embodiments, such method provides for a combination of these phenomena.

In some embodiments, such method results in an ability to administer Ketorolac for a longer duration than was previously achievable, without negative effect.

In some embodiments, such sustained dosage is attainable, due to reduced adverse effect, which in some aspects, may include reduced renal damage, reduced hepatic damage, and other side effects associated with the administration of sustained dosages in a given subject.

In some embodiments, such lowered or sustained dosage results in ultimate delivery of complete analgesia, which was not previously attainable in said subject. In some embodiments, such optimal dosage may be obtained faster and in a smaller volume in a subject, than was attainable with other Ketorolac compositions formulated for oral, nasal, or intravenous delivery, etc.

In some embodiments, a more concentrated fluid composition of Ketorolac is achieved by formulating the concentrated Ketorolac containing small volume sterile compositions as herein described, to be ethanol free and sodium chloride free and in some embodiments, such composition is significantly more stable over time than identical compositions containing ethanol and sodium chloride or compositions which are ethanol and sodium chloride free.

It is to be understood that reference to "Ketorolac" includes Ketorolac tromethamine and other pharmaceutically acceptable forms.

This invention provides a unit dosage form of Ketorolac or Ketorolac tromethamine in a sterile fluid composition formulated for continuous subcutaneous infusion for periods of at least up to 120 hours, wherein said Ketorolac or Ketorolac tromethamine is present at a dosage of between 40 mg/mL- 240 mg/mL. In some embodiments, the Ketorolac or Ketorolac tromethamine formulation is provided in a manner to deliver about 1 mL/day volumetric delivery. In some embodiments, the Ketorolac or Ketorolac tromethamine formulation is provided in a manner to deliver about 0.5 - 5 mL/day volumetric delivery.

In some embodiments, the subject is treated with a lower dosage for a sustained period of time, or in some embodiments, the dosage is titrated downward over time.

This invention provides, in some aspects, a selectively activatable body-worn infusion -pump assembly comprising a sealed prefilled drug-reservoir containing the unit dosage form of Ketorolac or Ketorolac tromethamine in a sterile fluid composition formulated for subcutaneous infusion as herein described.

In some embodiments, the invention provides a method of providing analgesia in a subject in need thereof, wherein said subject is administered the unit dosage form of Ketorolac in a sterile fluid composition formulated for sustained subcutaneous delivery, as herein described.

In some embodiments, the invention provides a method of reducing irritation, inflammation or a combination thereof in a subject in need thereof, wherein said subject is administered the unit dosage form of Ketorolac in a sterile fluid composition formulated for sustained subcutaneous delivery, as herein described.

### BRIEF DESCRIPTOIN OF THE DRA WINGS

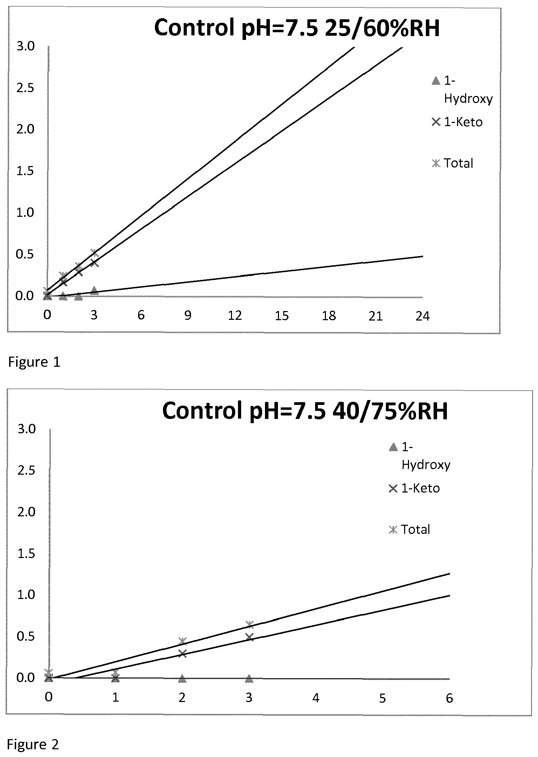

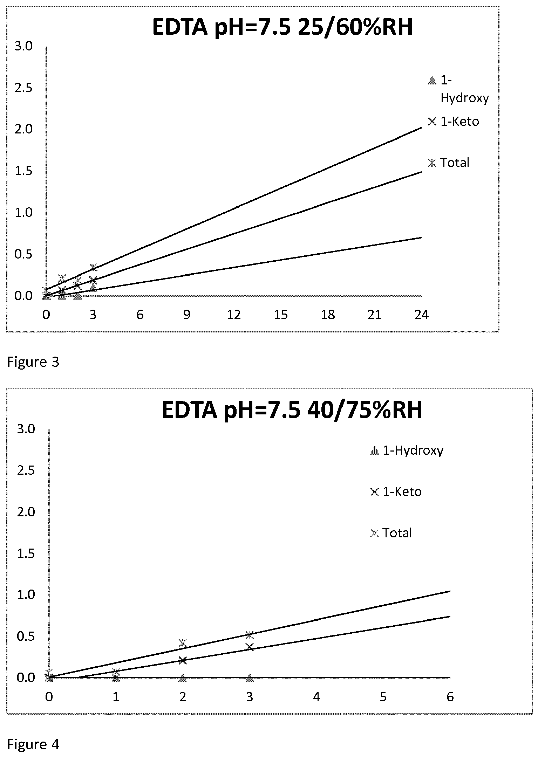

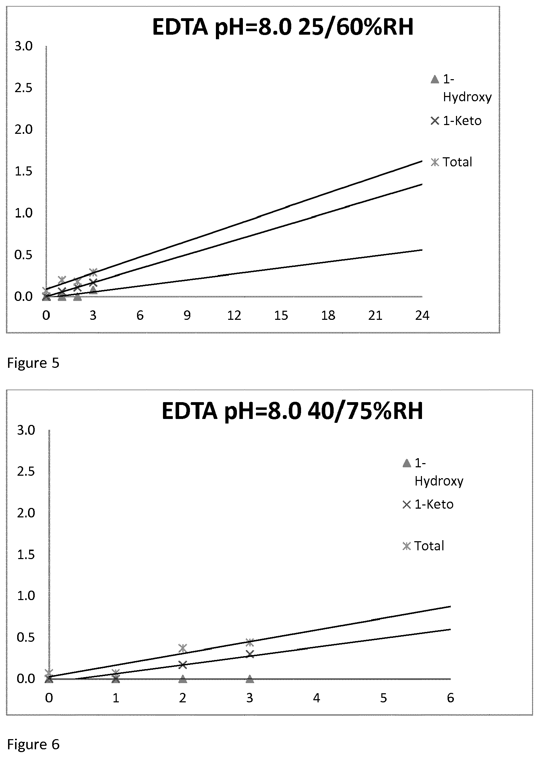

Figures 1-6 plot the relative impurities content for the 1-hydroxy and 1-keto impurities and total impurities over time in control samples, versus samples containing EDTA and being at a pH of 7.5 or 8, at 25ºC/60%RH versus at 40ºC/75%RH.

### DETAILED DESCRIPTION OF THE INVENTION

This invention aims to provide a Ketorolac treatment for patients in need of same, which is delivered over time via continuous subcutaneous administration, which Ketorolac is formulated for continuous subcutaneous delivery in a sterile, single use fluid composition, providing, inter alia, analgesia, an antipyretic or anti-inflammatory effect to said subject, and in some embodiments, providing for reduced adverse effects than seen to date in subjects receiving alternate formulations of Ketorolac.

In some embodiments, Ketorolac is provided in a single use container that provides therapy for one or several days at a time, and where such container can supply Ketorolac at a rate of 40-240 mg/ml, in a delivery volume of 1-5 mL per day. In some embodiments, the Ketorolac is provided as two or more single use containers that provide such therapy.

Surprisingly, providing Ketorolac by continuous subcutaneous injection/infusion in the formulation and as contained in the devices as described herein provided for a product with greater long-term stability/shelf life as typically seen in other formulations for parenteral delivery.

In some aspects of the invention, there is provided a unit dosage form of Ketorolac comprising an aseptically filled drug reservoir that may be stored unopened at room temperature for at least 18 months, or in some embodiments, for at least 24 months, or in some embodiments for at least 36 months, or in some embodiments, for any range within 18 and 36 months, or in some embodiments, for longer periods of time..

In some aspects of the invention, there is provided a unit dosage form of Ketorolac comprising an aseptically filled drug reservoir suitable for continuous infusion for a period for about 24 hours or in some embodiments, for more than 24 hours. In some embodiments, there is provided a unit dosage form of Ketorolac comprising an aseptically filled drug reservoir suitable for continuous infusion for a period of from 24 hours to 48 hours, or from 36 hours to 72 hours, or from about 24 hours to about 5 days.

In some aspects of the invention, there is provided a unit dosage form of Ketorolac comprising an aseptically filled drug reservoir suitable for continuous infusion, where the Ketorolac formulation comprises 0.5-5 mg/mL, or in some embodiments, 1-3 mg/mL, or in some embodiments, about 2-3 mg/mL, or in some embodiments, about 2 mg/mL sodium EDTA, which in some embodiments is further characterized as being sodium chloride free, or in some embodiments, ethanol free, or in some embodiments, characterized by a pH of greater than 7.5, or in some embodiments, having a pH from about 7.5 - 8.5, or in some embodiments, having a pH from about 7.5 - 8.3, or in some embodiments, having a pH from about 7.8 - 8.3.

In some aspects of the invention, there is provided a unit dosage form of Ketorolac comprising an aseptically filled drug reservoir suitable for continuous infusion, where the Ketorolac formulation is characterized by a tonicity of between 160 and 270 mOsmol/kg for a formulation comprising 45 mg/mL Ketorolac and in some embodiments, the tonicity is between 360 and 500 mOsmol/kg for a formulation comprising 90 mg/mL Ketorolac. In some aspects of the invention, there is provided a unit dosage form of Ketorolac comprising an aseptically filled drug reservoir suitable for continuous infusion, where the aseptically filled drug reservoir is characterized by a diameter at least 3 times its height, or in some embodiments, from at least 3 times to about 50 times its height, and in some embodiments, having a volume of 1 mL to 3 mL or in some embodiments, having a volume of from about 1 mL to 5 mL.

In some aspects of the invention, there is provided a unit dosage form of Ketorolac comprising an aseptically filled drug reservoir suitable for continuous infusion, where the surfaces of said reservoir walls comprises a plastic from the group including COP (cyclic olefin polymer) and COC (cyclic olefin copolymer).

In some aspects, there is provided a unit dosage form of Ketorolac comprising an aseptically filled drug reservoir where no more than 3% of the Ketorolac contained therein degrades over a period of 3 months when stored at 25 degrees and 60% humidity and in some embodiments, no more than 3% of the Ketorolac contained therein degrades over a period of 3 months when stored at 40 degrees and 75% humidity.

Example 2, Figures 1-6 show that surprisingly, despite the absence of ethanol, a stable formulation may be attained, which would show room temperature stability for more than 12 months, and production of far fewer impurities, when the Ketorolac formulations for infusion as described herein contain only EDTA as a preservative, and such results may be further improved if the formulation pH is more than 7.5, or between pH 7.55 and 8.0.

In some aspects, the formulation stability at higher temperatures would be similarly sustained, when evaluating the Ketorolac formulations of this invention, when the formulations are alcohol-free and contain only the single preservative EDTA, when the formulation pH is more than 7.5, or between pH 7.55 and 8.0.

In some aspects of this invention, there is provided an aseptically filled, single-use container for delivery via pump the single-use container comprising a parenteral formulation of Ketorolac for continuous subcutaneous delivery to a subject in need of such treatment, which provides, in some embodiments, for an ability to deliver a loading dose of Ketorolac injected at the start of the infusion, whereby such bolus profile provides for efficacious blood levels quickly while also incurring a lower risk of side effects or reduced side effects and same is an added advantage to the formulations of this invention in addition to the enhanced stability, as described herein. In some aspects, such side effects may be further adversely affected when formulation stability begins to decline and in some aspects, the formulations of the present invention specifically prevent or reduce same.

The Ketorolac as used in the compositions, devices and methods of the present invention are conveniently prepared by methods the same as or analogous to those described in U.S. Pat. No. United States Patent 4,089,969, 5,532,38; 6,191,285; 6,197,976; 6,323,344; each of which is hereby incorporated by reference in its entirety, or any other appropriate method, as will be appreciated by the skilled artisan.

The present invention extends to compositions, devices and methods of using physiologically acceptable salts of Ketorolac, as well as non-physiologically acceptable salts of Ketorolac that may be used in the preparation of the pharmacologically active compositions of the invention.

The term "pharmaceutically acceptable salt" refers to a salt of Ketorolac with an inorganic base, organic base, inorganic acid, organic acid, or basic or acidic amino acid. Salts of inorganic bases can be, for example, salts of alkali metals such as sodium or potassium; alkaline earth metals such as calcium and magnesium or aluminum; and ammonia. Salts of organic bases can be, for example, salts trimethylamine, triethylamine, pyridine, picoline, ethanolamine, diethanolamine, tromethamine, and triethanolamine. Salts of inorganic acids can be, for example, salts of hydrochloric acid, hydroboric acid, nitric acid, sulfuric acid, and phosphoric acid. Salts of organic acids can be, for example, salts of formic acid, acetic acid, trifluoroacetic acid, fumaric acid, oxalic acid, lactic acid, tartaric acid, maleic acid, citric acid, succinic acid, malic acid, methanesulfonic acid, benzenesulfonic acid, and p-toluenesulfonic acid. Salts of basic amino acids can be, for example, salts of arginine, lysine and ornithine. Salts of acidic amino acids can include, for example, salts of aspartic acid and glutamic acid. Quaternary ammonium salts can be formed, for example, by reaction with lower alkyl halides, such as methyl, ethyl, propyl, and butyl chlorides, bromides, and iodides, with dialkyl sulphates, with long chain halides, such as decyl, lauryl, myristyl, and stearyl chlorides, bromides, and iodides, and with aralkyl halides, such as benzyl and phenethyl bromides .

The present invention specifically envisions devices and methods of using same for the continuous subcutaneous delivery to a subject of a Ketorolac-containing formulation as herein described, including, in some embodiments, providing for a bolus administration of same followed by such continuous subcutaneous delivery. In some aspects, the devices envisioned will comprise some or all of the elements as described in U.S. Patent Number 8,834,454, U.S. Patent Application Publication Number 2009-0093772, U.S. Patent Application Publication Number 2014/0148761, each of which is fully incorporated herein by reference.

In some embodiments, reference to the compositions, kits and methods/uses of the invention, which refer to the term "comprise" or "comprising" also encompasses "consist of" or "consisting of".

In some embodiments, the unit dosage forms, compositions, kits and methods consist essentially of Ketorolac, in a formulation, whereby the term "consist essentially of" specifically excludes an additional active ingredient with anti-pyretic, or anti-inflammatory activity. In some embodiments, the term "consist essentially of" specifically excludes an additional active ingredient with anti-oxidant activity.

In some embodiments, the unit dosage forms, compositions, kits and methods comprise or make use of ketorolac. In some embodiments, the dosage forms, compositions, kits and methods consist or make use of Ketorolac. In some embodiments, the dosage forms, compositions, kits and methods consist essentially of or make use essentially of Ketorolac.

In some embodiments, the term "comprise" refers to the inclusion of the indicated active agent, such as Ketorolac, as well as inclusion of other active agents, and pharmaceutically acceptable carriers, excipients, emollients, antioxidants, stabilizers, etc., as are known in the pharmaceutical industry.

In some embodiments, the compositions of this invention will consist essentially of an active Ketorolac ingredient. In some embodiments, the term "consisting essentially of" refers to a composition whose only active ingredient of a particular class of agents, is the indicated active ingredient, i.e. the only active NSAID is Ketorolac, however, other compounds may be included which are involved directly in the therapeutic effect of the indicated active ingredient. In some embodiments, with reference to the compositions of this invention, when referring to a composition consisting essentially of an active Ketorolac ingredient, such reference specifically excludes the incorporation of any other NSAID in the composition.

"Pharmaceutical composition" or "composition" means a composition containing one or more drugs or prodrugs, and optionally one or more excipients, as well as any product which results, directly or indirectly, from combination, complexation or aggregation of any two or more of the excipients and/or the drug or prodrug, or from dissociation of one or more of the excipients and/or drug and/or prodrug, or from other types of reactions or interactions of one or more of the excipients and/or drug and/or prodrug. Accordingly, the pharmaceutical composition of the present invention encompasses any composition obtainable by admixing a carrier-linked Ketorolac of the present invention and a pharmaceutically acceptable excipient.

The term "excipient" refers to a diluent, adjuvant, or vehicle with which the carrier-linked Ketorolac is administered. Such pharmaceutical excipient can be sterile liquids, such as water and oils. In some aspects, saline and aqueous dextrose are preferred excipients when the pharmaceutical composition is administered intravenously.

Saline solutions and aqueous dextrose and glycerol solutions are preferably employed as liquid excipients for injectable/infusion solutions.

The composition, if desired, can also contain minor amounts of wetting or emulsifying agents, pH buffering agents, like, for example, acetate, succinate, tris, carbonate, phosphate, HEPES (4-(2- hydroxyethyl)-1-piperazineethanesulfonic acid), MES (2-(N-morpholino)ethanesulfonic acid), or can contain detergents, like Tween, poloxamers, poloxamines, CHAPS, Igepal, or amino acids like, for example, glycine, lysine, or histidine.

Examples of suitable pharmaceutical excipients are described in "Remington's Pharmaceutical Sciences" by E.W. Martin. Such compositions will contain a therapeutically effective amount of Ketorolac in the form of at least one carrier- linked Ketorolac prodrug of the present invention, preferably in purified form, together with a suitable amount of excipient so as to provide the form for proper administration to the patient. The formulation should suit the mode of administration.

The term "pharmaceutically acceptable" means approved by a regulatory agency such as the EMA (Europe) and/or the FDA (US) and/or any other national or regional regulatory agency for use in animals, preferably in humans.

In some aspects, the composition will comprise an anti-oxidant. In some embodiments, the anti-oxidant is selected for instance from acids and theirs salts, vitamins and derivatives, amino acids, sulfites or free phenolic radical scavengers. When the antioxidant is an acid or a salt thereof, it may be selected for instance from ascorbic acid or its salts such as sodium ascorbate, isoascorbic acid or its salts such as sodium isoascorbate, citric acid or its salts such as sodium citrate, lactic acid or malic acid.

When the antioxidant is a vitamin or a vitamin derivative, it may be selected for instance from tocopherol (vitamin E), riboflavin (vitamin B2), tocopherol-PEG-succinate (vitamin derivative) or trolox (vitamin derivative).

When the antioxidant is an amino acid, it may be selected for instance from cysteine, tryptophane, histidine, selenocysteine, N-acetyl cysteine, taurine, glutathione or glutathione-glutathione.

When the antioxidant is a sulfite, it may be selected for instance from sodium sulfite or sodium metabisulfite.

When the antioxidant is a phenolic free radical scavenger, it may be selected for instance from butylated hydroxy toluene, butyl hydroxyl anisole or cinnamic acid.

In some aspects, the amount of Ketorolac or its derivative, or a pharmaceutically acceptable salt thereof, which is provided in a medication or kit according to the invention is sufficient to achieve the desired effect, which in some aspects, will depend on the concentration of the compound used, and the weight and condition of the patient.

For example, and providing guidance for the envisioned formulations and kits and applications in the methods of this invention, a daily dose may be in the range supplying Ketorolac at a rate of about 80 - 90 mg per day in normal adults, or a daily dose may be in the range supplying Ketorolac at a rate of about 40 - 50 mg per day in elderly or underweight adults, or those with renal impairment.

For example, a subcutaneous dose may be provided in a unit dosage form administered continuously, supplying a daily dosage of about 80 - 90 mg in 1 mL, or 40-50 mg in 1mL, or 120-240 mg in 2-5mL, which may conveniently be administered as an infusion, for example, using a pump compatible with the single-use container. For example, a subcutaneous dose may be provided in a unit dosage form administered continuously, supplying a daily dosage of about 80 - 90 mg in 0.5 - 3 mL, or 40-50 mg in 0.5 - 5 mL, or 120-240 mg in 0.5 - 5 mL, which may conveniently be administered as an infusion, for example, using a pump compatible with the single-use container.

In some aspects, a subcutaneous dose may be provided in a unit dosage form supplying a daily dosage of from 40-240 mg, which dosage may still further be increased as needed, or in some embodiments, decreased as needed. In some embodiments changes in dosage may be implemented over a much prolonged period without ill effect, or in some embodiments, a lower dosage is necessary to achieve the desired therapeutic effect.

In some aspects, infusion fluids suitable for this purpose contain, for example, a delivery dosage of from 40-240 mg per day, provided in a minimal volume, which does not exceed 10 mL, and in some aspects is in a volume that is about 0.5 - 5 mL, and in some aspects is in a volume that is about 1-3 mL, or less.

In the manufacture of a medicament according to the invention, hereinafter referred to as a "formulation," Ketorolac and/or its derivatives, and/or pharmaceutically acceptable salts thereof, may be admixed with, inter alia, an acceptable carrier. The carrier must, of course, be acceptable in the sense of being compatible with any other ingredients in the formulation and must not be deleterious to the subject. One or more of Ketorolac or its derivatives, or pharmaceutically acceptable salts thereof, may be incorporated in the formulations of the invention, which may be prepared by any of the well-known techniques of pharmacy for admixing the components.

As will be appreciated by the skilled artisan, the terms "subject" and "patient" are used interchangeable and refer to any subject in need of or benefitting in any way from the described treatment/administration protocol/method of kit provided by the instant invention.

This invention provides, in some aspects, a selectively activatable body-worn infusion -pump assembly comprising a sealed prefilled drug-reservoir containing the unit dosage form of Ketorolac in a sterile fluid composition formulated for subcutaneous infusion as herein described.

In some aspects, this invention provides an automatic infusion device comprising a sealed prefilled drug-reservoir containing the unit dosage form of Ketorolac in a sterile fluid composition, formulated for continuous subcutaneous infusion as herein described.

In some embodiments, the term "automatic infusion device" refers to a device that enables an individual (also referred to herein as a user or a patient or subject) to self-administer a dosage of a substance, such as a liquid medication, wherein the device differs from a standard syringe by the inclusion of a mechanism for automatically delivering the medication to the individual by infusion when the mechanism is engaged.

In some aspects, as will be appreciated by the skilled artisan, any appropriate automatic infusion device may be used, for example, as described in U.S. Pat. Nos. 3,910,260; 4,004,577; 4,689,042; 4,755,169; 4,795,433; 3,941,130; 4,261,358; 5,085,642; 5,092,843; 5,102,393; 5,267,963; 6,149,626; 6,270,479; 8,679,061 and 6,371,939, each of which is incorporated by reference herein in its entirety.

In some embodiments, PK-PD modeling suggests that blood level of 5 mcg/mL represents an approximate safety threshold for the drug. With the available intermittent/bolus dosing regimen for Ketorolac, high spikes in blood levels have been reported. Surprisingly, using the device/formulation of Ketorolac and via the methods described herein, blood level fluctuations, in some embodiments are reduced or abrogated, and in other embodiments, exceeding a 5 mcg/mL threshold is without significant deleterious effect. In some embodiments, per the PK-PD analysis described further herein below, the use of a body-worn infusion Pump for the delivery of Ketorolac improves the side-effect profile of therapies with this drug by e.g. reducing the incidence or severity of the side-effects noted with such use.

In some embodiments, the invention provides Ketorolac formulations and formulated for use with infusion devices as herein described, which provide greater shelf-life in terms of their long-term stability, which in some aspects, are further characterized by additional advantages as described herein, for example, including dose-sparing effects as described herein.

In some embodiments, the provides Ketorolac formulations and formulated for use with infusion devices as herein described, which provide greater shelf-life in terms of their long-term stability, which in some aspects, are further characterized by additional advantages as described herein, for example, including a formulation with enhanced stability even once the container is opened.

In some embodiments, the invention provides Ketorolac formulations and formulated for use with infusion devices as herein described, which are much more concentrated in terms of the Ketorolac concentration as compared to Ketorolac formulations comprising the state of the art. Surprisingly, such highly concentrated compositions are stable and provide for greater long-term storage than other existing Ketorolac formulations. In some embodiments, use of higher Ketorolac concentrations and delivery of smaller volumes provides the opportunity for dose sparing effects as herein described.

In some embodiments, the invention provides Ketorolac formulations and formulated for use with infusion devices as herein described, which include use of same in a pre-programmed pump containing device as described, such that a loading dose and the basal dose are provided to a subject, with the controls for same provided in a single unit. In some embodiments, such arrangement provides for controlled delivery without human intervention required to shift from bolus to basal level delivery, providing for the bolus delivery to quickly reach efficacious levels, without increasing the risk of side effects in the subject thus treated. In some aspects the enhanced stability of the instant formulations are particularly helpful in this context and provide unexpected advantages with respect to same.

In some embodiments, the Ketorolac as formulated in addition, delivers a lower dosage than alternate formulations of Ketorolac, or in some embodiments, the subject is treated with a lower dosage for a sustained period of time than is typically administered in patients receiving Ketorolac therapy, or in some embodiments, the dosage is titrated downward over time, which in some embodiments, is due to reduced adverse effects than what is typically experienced with other Ketorolac treatment regimens in affected subjects. In some aspects, this advantage is in addition to the added formulation stability as described herein.

Surprisingly, providing Ketorolac by continuous subcutaneous infusion is associated not only with optimal analgesia, anti-pyretic effects and reduced severe irritation/inflammation in the subject, but the same may be accomplished using lower drug dosages, and being accompanied by fewer adverse effects. Furthermore, surprisingly, the same is further associated with an improved responsiveness to Ketorolac. In some aspects, such improved performance is seen in the ability to provide sustained use of a relatively low dose, or in some embodiments, a slower time to increasing such dosage, or in some aspects, a titration downward of a dosage, or in some embodiments, a less frequent need for administration of Ketorolac, or in some embodiments, any combination of these effects.

In some aspects, the Ketorolac formulation provides for an added dose-sparing effect. In some aspects, the adult daily dosage may be provided as from 40 - 240 mg/day. In some aspects, the adult daily dosage typically used for normal weight, non-elderly patients is 120 mg/day, and a daily dosage 25% - 35% less than same can be provided with the formulations and delivery system of this invention. For example, in some embodiments, formulations providing a daily dosage of 80-90 mg/day are envisioned. In some aspects, the daily dosage for elderly, or underweight patients typically used is 60 mg/day, which as well may be reduced by from 25% - 35% less, by using the formulations and delivery systems of this invention. For example, in some embodiments, formulations providing a daily dosage of 40-45 mg/day are envisioned.

In some embodiments, a similar dose sparing effect is achievable in pediatric formulations, and the daily dosage will be titrated downward, accordingly, as will be appreciated by the skilled artisan, with the same ability to reduce such pediatric daily doseage by from 10-35% less than that typically in use via other administration routes/formulation protocols.

In some aspects, the invention provides Ketorolac formulations and formulated for use with infusion devices as herein described, which additionally increase the local tolerance of the subject to Ketorolac, which may provide additional advantages, such as, for example, treatment, the avoiding in situ precipitation when formulating more concentrated compositions; increasing the stability of the formulation and providing for an increased drug concentration allowing the reduction of the infusion volume.

In some aspects, this invention provides a kit comprising a formulation of Ketorolac for continuous subcutaneous delivery to a subject in need of such treatment, which provides analgesic effects, antipyretic effects or anti-inflammatory effects. In some aspects, such kits may provide multiple sterile formulations of Ketorolac for continuous subcutaneous delivery, which vary in terms of the dosage delivered of same.

In some aspects, such kits comprise a container, which may include a vial, for infusion, or in some embodiments, one or more prefilled drug-reservoirs containing the Ketorolac formulation comprising Ketorolac in a pharmaceutically acceptable carrier. In some aspects, the kits may further comprise instructions, such as a product insert or label, directing the user regarding proper administration and use of the formulation, or in some embodiments, instructions for making use of the formulations varying, for example in terms of Ketorolac dosage provided with said kit, which in some embodiments, provides instructions for titrating a dosage of same.

In some aspects, such kits may comprise a tubing set to connect the drug reservoir to the patient's cannula or catheter (also referred to herein as the means of administration), or in some embodiments, the means of administration is provided to the patient integrated with the container.

In some embodiments, the invention provides a method of reducing pain or providing analgesia in a subject in need thereof, wherein said subject is administered the unit dosage form of Ketorolac in a sterile fluid composition as herein described or in some embodiments, the subject is administered Ketorolac by making use of a kit as herein described.

In some embodiments, the invention provides a method of reducing irritation, inflammation or a combination thereof in a subject in need thereof, wherein said subject is administered the unit dosage form of Ketorolac in a sterile fluid composition as herein described or in some embodiments, the subject is administered Ketorolac by making use of a kit as herein described.

In some embodiments, the present invention overcomes previous restricted use of Keterolac in terms of dosages and duration of therapy allowed in treated subjects, providing sustained delivery via the formulations as described herein and via use of the selectively activatable body-worn infusion -pump assemblies comprising Ketorolac as described herein.

In some aspects the present invention overcomes previous limitations in terms of the existing dilute Ketorolac formulations and the inclusion of ethanol in same, by providing the ability for more concentrated, low ethanol or ethanol free formulations.

In some embodiments, such advantages, in turn, may provide for Ketorolac-containing compositions with greater stability, or in some embodiments, such advantages may comprise the ability to deliver a unit dosage container which is smaller, and provide for greater ease and flexibility with respect to delivery systems/devices containing same.

In some embodiments, the present invention overcomes previous limitations in terms of the existing Ketorolac formulations and the inclusion of ethanol in same, by obviating or reducing side effects, such as irritation and inflammation, or administration site pain as a result of or as contributed to by the inclusion of ethanol in such formulations.

In some embodiments, the present invention overcomes previous limitations in terms of the existing Ketorolac formulations, in providing analgesia over a more extended period of time. In some embodiments, treatment beyond 5 consecutive days is uniquely achievable using the embodied dosage forms, compositions and body-worn infusion pump assemblies containing same. In some aspects, the specific drug delivery assemblies envisioned for use with the embodied dosage forms and compositions as herein described allow for slim profile more convenient devices, in part due to the ability to prepare more concentrated stable Ketorolac containing compositions.

In some aspects, the present invention thus provides a concentrated, stable and low-ethanol or ethanol free formulation, which in some embodiments, is provided as part of a compact subcutaneous delivery system, allowing for safer and more tolerated Ketorolac delivery, with for example, safer dosing and delivery sustained over longer periods of time than currently possible.

In some aspects, the present invention promotes the ability to arrive at more concentrated Ketorolac containing formulations, and in some aspects, the present invention provides for highly precise, controlled delivery systems to ensure safe dosing of such concentrated formulations, promoting optimal Ketorolac delivery.

In some aspects, the Ketorolac-containing formulations of this invention, even when available in higher concentrations than previously envisioned nonetheless are well tolerated, without significant side effect.

All publications and patents mentioned herein are hereby incorporated by reference in their entirety as if each individual publication or patent was specifically and individually indicated to be incorporated by reference. In case of conflict, the present application, including any definitions herein, will control.

While specific embodiments of the subject invention have been discussed, the above specification is illustrative and not restrictive. Many variations of the invention will become apparent to those skilled in the art upon review of this specification and the claims below. The full scope of the invention should be determined by reference to the claims, along with their full scope of equivalents, and the specification, along with such variations.

### EXAMPLES

### KETOROLAC CONTAINING FORMULATIONS FOR SUBCUTANEOUS DELIVERY

### EXAMPLE 1

Embodied compositions are prepared containing the following:

| | |
|---|---|
| Keterolac tromethamine: | 60, 90 and 120 mg/mL and other various increments |
| Sodium ascorbate: | 0-2 mg/ml |
| Sodium phosphate buffer: | 0-2 mg/ml |
| Ethanol: | 0-20% |
| 1N HCl or 1N NaOH, USP: | As needed for pH adjustment [to achieve a neutral pH] |
| Water for injections: | As needed per batch size |

In some aspects, the formulation is a non-ethanolic formulation.

The formulation is sterile filtered, aseptically filled into pre-sterilized unit dose containers, and stoppered, prior to assembly within an integrated delivery system.

### PREPARATION OF AN EXEMPLIFIED KETOROLAC FORMULATION FOR CONTINUOUS INFUSION

### EXAMPLE 2

The design of a filled primary container for delivery by continuous subcutaneous infusion via body-worn infusion pump device is achieved by means of use of a container that is aseptically filled with a Ketorolac tromethamine formulation as described stoppered with an elastomeric septum and capped with a primary container cap.

The filled primary container is supplied as a pre-filled unit and can be provided in various appropriate at desired product strengths, for example, having a Ketorolac tromethamine concentration of 45 and 90 mg/mL. The fill volume of each unit is approximately 2.65 mL.

Toward this end, in 800 mL of water for injection was dissolved 2 g of Disodium EDTA dihydrate and 90 g of Ketorolac tromethamine, the pH was adjusted to pH 8.0 with 1N Sodium Hydroxide and water for injection was added to complete the 1 liter volume. The skilled artisan will appreciate that appropriate scale up of the amounts is envisioned, as well.

### EVALUATION OF PHYSICAL AND CHEMICAL STABILITY OF AN EXEMPLIFIED KETOROLAC FORMULATION FOR CONTINUOUS INFUSION

### EXAMPLE 3

A 90 mg/mL Ketorolac Tromethamine formulations was prepared as described hereinabove for Example 2. The formulation was stored at either 25°C / 60% relative humidity (RH) or at 40°C / 75%RH for 3 months, where conditions at 25°C / 60% relative humidity (RH) generally represents "room temperature" and conditions at 40°C / 75%RH represents a roughly 4-times Arrhenius acceleration factor. The latter conditions mimic storage/stability conditions encountered over 12 months at room temperature for the formulation.

Briefly, 90 mg/mL Ketorolac Tromethamine were prepared. The bulk solution was then filtered using 0.22 µm PVDF syringe filters, filled into the primary container at 2.65 mL fill volume/container, then sealed with septa using an insertion rig and the tests and schedule as outlined in **Table 1** and **Table 2** was pursued.

The testing parameters included, Physical Appearance via visual inspection for color and clarity; pH evaluation and UPLC analysis using standard methodology and equipment.

**Table 1: Test Methods for Drug Product Samples**

| **Test Codes** | **Sample Containers** | **Test** | **Test Methods** |
|---|---|---|---|
| A | **1** | Physical Appearance | Visual Inspection |
| B | | pH | pH meter |
| C | **2** | Assay (n=2) | UPLC analysis |
| D | | Related Substances (n=2) | UPLC analysis |

**Table 2: Stability Test Schedule**

| Conditions | Time points (months) | | | | | |
|---|---|---|---|---|---|---|
| | 0 | 1 | 2 | 3 | 6 | Reserve |
| 25°C/60% RH | A-D | A-D | A-D | A-D | A-D | |
| 40°C/75% RH | | A-D | A-D | A-D | A-D | |
| Total containers required per time point @ 25°C/60%RH | | 3 | 3 | 3 | 3 | 6 |
| Total vials required per time point @ -40°C/75%RH | | 3 | 3 | 3 | 3 | 6 |
| Total vials for T=0 | | | | | | 3 |
| Total number of vials for 25°C/60%RH | | | | | | 18 |
| Total number of vials for 40°C/75%RH | | | | | | 18 |
| Total | | | | | | 39 |

The results for physical appearance, pH, and water transmission rate evaluation are summarized in Table 3.

**Table 3: Summary Results for Physical Appearance, pH, and WVTR Evaluation**

| **Formulation** | **Storage Condition** | **Time Points (months)** | **Phys. App.** | **pH** | **WVTR (g)** |
|---|---|---|---|---|---|
| Ketorolac Tromethamine 90 mg/mL 2 mg/mL EDTA pH = 7.5 | 25ºC/60%RH | 0 | CSLY solution | 7.42 | N/A |
| | | 1 | CSLY solution | 7.48 | 0.003 |
| | | 2 | CSLY solution | 7.44 | 0.006 |
| | | 3 | CSLY solution | 7.51 | 0.009 |
| | | 6 | | | |
| | 40°C/75%RH | 1 | CSLY solution | 7.49 | 0.003 |
| | | 2 | CSLY solution | 7.51 | 0.014 |
| | | 3 | CY solution | 7.55 | 0.023 |
| | | 6 | | | |
| Ketorolac Tromethamine 90 mg/mL 2 mg/mL EDTA pH = 8.0 | 25ºC/60%RH | 0 | CSLY solution | 7.97 | N/A |
| | | 1 | CSLY solution | 7.99 | 0.003 |
| | | 2 | CSLY solution | 8.02 | 0.006 |
| | | 3 | CSLY solution | 8.03 | 0.009 |
| | | 6 | | | |
| | 40°C/75%RH | 1 | CSLY solution | 7.99 | 0.007 |
| | | 2 | CSLY solution | 8.04 | 0.014 |
| | | 3 | CY solution | 8.04 | 0.022 |
| | | 6 | | | |
| Ketorolac Tromethamine 90 mg/mL (Control) pH = 7.5 | 25ºC/60%RH | 0 | CSLY solution | 7.42 | N/A |
| | | 1 | CSLY solution | 7.48 | 0.003 |
| | | 2 | CSLY solution | 7.45 | 0.006 |
| | | 3 | CSLY solution | 7.49 | 0.009 |
| | | 6 | | | |
| | 40°C/75%RH | 1 | CSLY solution | 7.47 | 0.007 |
| | | 2 | CSLY solution | 7.51 | 0.014 |
| | | 3 | CY solution | 7.47 | 0.023 |
| | | 6 | | | |

The assay results and oxidative degradation impurities obtained are summarized in **Tables 4,5 and 6.** Figures 7-12 plot the results in terms of the listed impurities in the tables.

**Table 4: Summary Results for Assay and Related Substances Ketorolac 90 mg/mL with 2 mg/mL EDTA pH 7.5**

| **Formulation** | **Storage Concition** | **Time Points (months)** | **Replicate** | **Assay** | **Impurities (Area %)** | | | | | | |
|---|---|---|---|---|---|---|---|---|---|---|---|
| | | | | | **RRT 0,14** | **RRT 0.35** | **RRT 0.39** | **RRT 0.70** | **RRT 0.73 (1-Hydroxy)** | **RRT 1.40 (1-Keto)** | **Total Imp.** |
| | 25ºC/60%RH | 0 | 1 | 102.2 | < 0.05% | NR | ND | 0.06 | ND | ND | 0.06 |
| | | | 2 | 98.7 | < 0.05% | NR | ND | 0.07 | ND | ND | 0.07 |
| | | 1 | 1 | 100.0 | 0.08 | NR | < 0.05% | 0.06 | ND | 0.07 | 0.21 |
| | | | 2 | 99.4 | 0.08 | NR | < 0.05% | 0.07 | ND | 0.07 | 0.22 |
| | | 2 | 1 | 100.6 | % < 0.05% | NR | < % 0.05% | 0.06 | ND | 0.12 | 0.18 |
| Ketorolac | | | 2 | 100.5 | < 0.05% | NR | < 0.05% | 0.07 | ND | 0.12 | 0.19 |
| Tromethamine | | 3 | 1 | 100.6 | NR | 0.05 | NR | NR | 0.10 | 0.19 | 0.34 |
| 90 mg/mL | | | 2 | 100.9 | NR | 0.05 | NR | NR | 0.17 | 0.18 | 0.30 |
| 2 mg/mL | | 6 | 1 | | | | | | | | |
| EDTA pH = 7.5 | | | 2 | | | | | | | | |
| | 40°C/75%RH | 1 | 1 | 99.8 | 0.06 | NR | 0.08 | 0.07 | ND | 0.21 | 0.42 |
| | | | 2 | 97.8 | 0.06 | NR | 0.08 | 0.07 | ND | 0.21 | 0.42 |
| | | 2 | 1 | 99.9 | < 0.05% | NR | 0.09 | 0.06 | ND | 0.37 | 0.52 |
| | | | 2 | 99.8 | < 0.05% | NR | 0.09 | 0.06 | ND | 0.37 | 0.52 |
| | | 3 | 1 | 99.8 | NR | 0.10 | NR | NR | 0.07 | 0.55 | 0.72 |
| | | | 2 | 100.5 | NR | 0.08 | NR | NR | 0.07 | 0.57 | 0.72 |
| | | 6 | 1 | | | | | | | | |
| | | | 2 | | | | | | | | |

**Table 5: Summary Results for Assay and Related Substances Ketorolac 90 mg/mL with 2 mg/mL EDTA pH 8.0**

| **Formulation Storage** | **Storage Condition** | **Time Points (months)** | **Replicate** | **Assay** | **Impurites [Area %)** | | | | | | |
|---|---|---|---|---|---|---|---|---|---|---|---|
| | | | | | **RRT 0.14** | **RRT 0.35** | **RRT 0.39** | **RRT 0.70** | **RRT 0.73 (1-Hydroxy)** | **RRT 1.40 (1-Keto)** | **Total Imp.** |
| | 25ºC/60%RH | 0 | 1 | 98.7 | < 0.05% | NR | ND | 0.07 | ND | ND | 0.07 |
| | | | 2 | 98.2 | < 0.05% | NR | ND | 0.07 | ND | ND | 0.07 |
| | | 1 | 1 | 99.3 | 0.07 | NR | < 0.05% | 0.07 | ND | 0.06 | 0.20 |
| | | | 2 | 99.8 | 0.07 | NR | < 0.05% | 0.07 | ND | 0.06 | 0.20 |
| | | 2 | 1 | 101.5 | < 0.05% | NR | < 0.05% | 0.07 | ND | 0.11 | 0.18 |
| Ketorolac | | | 2 | 99.6 | < 0.05% | NR | < 0.05% | 0.07 | ND | 0.11 | 0.18 |
| Tromethamine | | 3 | 1 | 100.0 | NR | 0.04 | NR | NR | 0.08 | 0.17 | 0.29 |
| 90 mg/mL | | | 2 | 100.9 | NR | 0.04 | NR | NR | 0.08 | 0.17 | 0.29 |
| 2 mg/mL EDTA pH = | | 6 | 1 | | | | | | | | |
| 8.0 | | | 2 | | | | | | | | |
| | 40°C/75%RH | 1 | 1 | 98.8 | 0.06 | NR | 0.07 | 0.07 | ND | 0.17 | 0.37 |
| | | | 2 | 99.8 | 0.06 | NR | 0.07 | 0.07 | ND | 0.17 | 0.37 |
| | | 2 | 1 | 99.7 | < 0.05% | NR | 0.07 | 0.07 | ND | 0.31 | 0.45 |
| | | | 2 | 99.8 | < 0.05% | NR | 0.07 | 0.07 | ND | 0.30 | 0.44 |
| | | 3 | 1 | 100.1 | NR | 0.08 | NR | NR | 0.07 | 0.47 | 0.62 |
| | | | 2 | 101.4 | NR | 0.07 | NR | NR | 0.08 | 0.47 | 0.62 |
| | | 6 | 1 | | | | | | | | |
| | | | 2 | | | | | | | | |

**Table 6: Summary Results for Assay and Related Substances Ketorolac 90 mg/mL pH 7.5 - Control**

| **Formutation^{.}** | **Storage Condition** | **Time Points (months)** | **Replicate** | **Assay** | **Im**p**urites** | | | | | | |
|---|---|---|---|---|---|---|---|---|---|---|---|
| | | | | | **RRT 0.14** | **RRT 0.35** | **RRT 0.39** | **RRT 0.70** | **RRT 0 73 (1-Hydroxy)** | **RRT 1.40 (1-Keto)** | **Total Imp.** |
| | 25ºC/60%RH | 0 | 1 | 95.9 | ND | NR | ND | 0.06 | ND | ND | 0.06 |
| | | | 2 | 99.7 | ND | NR | ND | 0.07 | ND | ND | 0.07 |
| | | 1 | 1 | 100.4 | < 0.05% | NR | < 0.05% | 0.07 | ND | 0.17 | 0.24 |
| | | | 2 | 100.4 | ND | NR | 0.05% | 0.07 | ND | 0.17 | 0.24 |
| | | 2 | 1 | 98.6 | ND | NR | 0.05% | 0.07 | ND | 0.29 | 0.36 |
| | | | 2 | 101.3 | ND | NR | < 0.05% | 0.07 | ND | 0.29 | 0.36 |
| | | 3 | 1 | 101.1 | NR | 0.05 | NR | NR | 0.07 | 0.40 | 0.52 |
| Ketorolac | | | 2 | 100.7 | NR | 0.05 | NR | NR | 0.07 | 0.43 | 0.55 |
| Tromethamine | | 6 | 1 | | | | | | | | |
| 90 mg/mL (Control) | | | 2 | | | | | | | | |
| pH = 7.5 | 40°C/75%RH | 1 | 1 | 100.5 | < 0.05% | NR | 0.08 | 0.07 | ND | 0.29 | 0.44 |
| | | | 2 | 99.0 | < 0.05% | NR | 0.08 | 0.07 | ND | 0.30 | 0.45 |
| | | 2 | 1 | 99.5 | < 0.05% | NR | 0.09 | 0.06 | ND | 0.50 | 0.65 |
| | | | 2 | 100.1 | < 0.05% | NR | 0.09 | 0.06 | ND | 0.50 | 0.65 |
| | | 3 | 1 | 100.0 | NR | 0.09 | NR | NR | 0.07 | 0.70 | 0.86 |
| | | | 2 | 100.6 | NR | 0.08 | NR | NR | 0.07 | 0.72 | 0.87 |
| | | 6 | 1 | | | | | | | | |
| | | | 2 | | | | | | | | |

The results indicate that the pH remains consistent throughout the study for formulations at both storage conditions, as does the percent assay for all formulations.

The impurities profile for the formulations exhibited a similar trend, where total impurities exhibited a trending upward at a slightly higher rate for samples stored at 40°C/75%RH as compared to samples stored at 25°C/60%RH and the 1-keto impurity was found to be the major contributor to the total impurity increase.

Surprisingly, the formulation of 90 mg/mL Ketorolac Tromethamine with 2 mg/mL EDTA at pH 8.0 out-performed comparable formulations with reasonably similar pH values tested.

It will be apparent to those skilled in the art that various modifications and variations can be made to the compositions and processes of this invention. Thus, it is intended that the present invention cover such modifications and variations, provided they come within the scope of the appended claims and their equivalents. The disclosure of all publications cited above is expressly incorporated herein by reference in their entirety to the same extent as if each were incorporated by reference individually.

## Claims

1. A unit dosage form of Ketorolac in a sterile fluid composition formulated for continuous subcutaneous delivery via body-worn infusion pump assembly, wherein said Ketorolac is present at a concentration of between **40** mg/mL and **240** mg/mL formulated for single use delivery in a volume not to exceed 1-10 mL, wherein said formulation is ethanol-free.

2. The unit dosage form of claim 1, wherein said composition is formulated for delivery of a daily dosage of Ketorolac from between about **50** - **120** mg/day.

3. The unit dosage form of claim 1, wherein said Ketorolac is present at a concentration of between **120** mg/mL and **240** mg/mL.

4. The unit dosage form of claim 1, wherein said composition provides for a maximal volume of infusion which does not exceed 3 mL for single use.

5. The unit dosage form of claim 4, wherein said composition comprises Disodium EDTA dihydrate as the preservative in said dosage form.

6. The unit dosage form of claim 5, wherein said composition is at a pH of about from 7.6 to about 8.0.

7. The unit dosage form of claim 1, wherein said composition provides for a maximal volume of infusion which does not exceed 1 mL for single use.

8. The unit dosage form of claim 1, wherein said Ketorolac is Ketorolac tromethamine.

9. A selectively activatable body-worn infusion-pump assembly comprising a sealed prefilled drug-reservoir containing the unit dosage form of claim 1.

10. A sterile fluid composition for continuous subcutaneous delivery via body-worn infusion pump assembly, said composition comprising Ketorolac at a concentration of between **40** mg/mL and **240** mg/mL in a volume not to exceed 1-5 mL such as in a volume that does not exceed 3 mL or in a volume that does not exceed 1 mL, wherein said sterile fluid composition is ethanol-free.

11. The sterile fluid composition of claim 10, wherein said composition comprises Disodium EDTA dihydrate as the preservative in said dosage form.

12. The sterile fluid composition of claim 10, wherein said composition is at a pH of about 8.0.

13. A unit dosage form of Ketorolac in a sterile fluid composition formulated for continuous subcutaneous delivery via body-worn infusion pump assembly, wherein said Ketorolac is present at a concentration of between **40** mg/mL and **240** mg/mL formulated for single use delivery in a volume not to exceed 1-10 mL, wherein said composition is alcohol free, and stable for a period of at least 12 months at room temperature.

14. The unit dosage form of claim 13, wherein said composition contains only a single preservative, which preservative is EDTA.

15. Ketorolac for use in a method of providing analgesia, anti-pyretic effects or reducing pain, or reducing administration site irritation, inflammation or a combination thereof, in a subject in need thereof, wherein said subject is administered Ketorolac in a sterile fluid composition formulated for continuous subcutaneous delivery, wherein said Ketorolac is present at a dosage of between **40** mg/mL and **240** mg/mL and wherein said composition is alcohol free, and stable for a period of at least 12 months at room temperature.

16. Ketorolac for the use of claim 15, wherein said Ketorolac is Ketorolac tromethamine.

17. Ketorolac for the use of claim 15, wherein said method provides for reducing a dosage of Ketorolac over time.

18. Ketorolac for the use of claim 15, wherein said method provides for sustaining a given dosage of Ketorolac over a prolonged period of time.

19. Ketorolac for the use of claim 15, wherein said method provides for reduced adverse effects typically accompanying Ketorolac administration by other routes of administration.

20. Ketorolac for the use of claim 19, wherein said adverse effects comprise gastrointestinal bleeding, inhibition of platelet function, renal impairment, or a combination thereof.

21. Ketorolac for the use of claim 15, wherein said method makes use of a selectively activatable body-worn infusion -pump assembly comprising a sealed prefilled drug-reservoir containing a unit dosage form comprising Ketorolac to administer Ketorolac to said subject.

22. Ketorolac for the use of claim 21, wherein said Ketorolac is formulated for single use delivery in a volume not to exceed 2-5 mL, such as in a volume not to exceed 3 mL or in a volume not to exceed 1 mL.

23. Ketorolac for the use of claim 15 wherein said subject is administered Ketorolac over a period of time of more than 5 consecutive days.
